# EUROPEAN PATENT APPLICATION

(11) **EP 1 717 587 A1**
(43) Date of publication of application: **02.11.2006**
(21) Application number: 05709910.3
(22) Date of filing: 08.02.2005
(51) Int. Cl.: G01N 33/68, G01N 27/447, G01N 27/62, C12Q 1/68, C12N 15/09, C12P 21/02

(54) **METHOD AND KIT FOR IDENTIFYING THE FUNCTION OF GENE PRODUCT AND IDENTIFYING BINDING SUBSTANCE**

(30) Priority: 16.02.2004 JP 2004038647
(71) Applicant: Human Metabolome Technologies, Inc., Tsuruoka-shi, Yamagata 9970015 (JP)
(72) Inventor: TOMITA, Masaru, Tsuruoka Town Campus, Keio Univ., Tsuruoka-shi, Yamagata 9970035 (JP); ITOH, Aya, Tsuruoka Town Campus, Keio Univ., Tsuruoka-shi, Yamagata 9970035 (JP); SOGA, Tomoyoshi, Tsuruoka Town Campus, Keio Univ., Tsuruoka-shi, Yamagata 9970035 (JP)
(74) Representative: Dehmel, Albrecht
(86) International application number: PCT/JP2005/001858
(87) International publication number: WO 2005/078449

(57) **Abstract**

[Problems to be Solved] An objective of the present invention is to provide methods and kits for identifying the function of a functionally unknown gene product, and methods and kits for identifying a binding substance, which are widely applicable to numerous organism species.

[Means to Solve the Problems] At least one gene product is added to a compound cocktail such as a metabolic compound cocktail containing all of the metabolic substances, coenzymes, and such involved in a certain metabolic system, the mixture is reacted, and then a change occurred in the compound cocktail is detected, thereby making it possible to identify the function of the gene product or a substance that binds thereto.

## Description

### Technical Field

The present invention relates to methods and kits for identifying the function of gene products with unknown function and methods and kits for identifying binding substances of the gene products.

### Background Art

A variety of genetic techniques are employed to verify the function of gene products. These include, for example, expression of antisense RNA, RNAi, or a dominant negative (dn)-type gene product in cultured cells or animals, disruption of a target gene to be analyzed by knocking out the gene, and forced expression of the gene using vectors for forced expression such as viral vectors and plasmid vectors, to examine the effect of the gene manipulation. In these cases, to examine the effect of the gene manipulation, one just has to compare the phenotypes in the presence or absence of the gene manipulation.

For example, histological analyses for examining a change in morphology or expression of a marker (such as observation of tissue sections or immunostaining), biochemical analyses for examining a change in biochemical activity (such as assays for enzyme activities of the extract), or molecular biological analyses for examining a change in gene expression (such as differential display), can be done (see, for example, Non-Patent Document 1).
[Non-Patent Document 1] Andersson U., Levander F. and Radstrom P., "Trehalose-6-phosphate phosphorylase is part of a novel metabolic pathway for Trehalose utilization in Lactococcus lactis", Journal of Biological Chemistry (USA), 276 (46), 42707-42713 (2001)).

### Disclosure of the Invention

### Problems to be Solved by the Invention

However, since systems employing cells or animals are very complicated, it is not easy to examine the effect of gene manipulations. In addition, gene manipulations described above can only be applied to a limited biological species.

Accordingly, an objective of the present invention is to provide methods and kits for identifying the function of gene products with unknown function and to provide methods and kits for identifying the binding substances of the gene products, which are widely applicable to a wide variety of biological species.

### Means to Solve the Problems

The methods of the present invention for identifying the function of a gene product include adding at least one gene product to a compound cocktail, reacting the mixture, detecting changes occurred in the compound cocktail, and thereby identifying the function of the gene product. In one embodiment, the methods of the present invention for identifying the function of a gene product comprises the steps of: adding at least one gene product to a compound cocktail; incubating the compound cocktail; removing the gene product from the compound cocktail; and detecting changes in the compounds contained in the compound cocktail.

The methods of the present invention for identifying the function of a gene product may further comprise the step of obtaining at least one gene product by expressing at least one gene encoding the gene product.

In the methods of the present invention, any means used for purification of substances including, ultrafiltration, column chromatography, salting out, solvent precipitation, solvent extraction, distillation, immunoprecipitation, SDS-polyacrylamide gel electrophoresis, isoelectric electrophoresis, dialysis, and recrystallization may be used for removing contaminants (for example, proteins such as the gene product added) from the compound cocktail.

Furthermore, in the present invention, detection of changes in compounds can be achieved by the steps of: (a) measuring the amount of each compound comprised in the compound cocktail with which the gene product is reacted; (b) measuring the amount of each compound comprised in the compound cocktail reacted under the same conditions, but without the gene product; and (c) comparing the amount of compound determined in steps (a) and (b), thereby identifying a compound that changed in quantity. Analytical instruments for identifying and quantifying the above compounds include capillary electrophoresis-mass spectrometer (CE/MS), liquid chromatography-mass spectrometer (LC/MS), gas chromatography-mass spectrometer (GC/MS), Fourier transform ion cyclotron resonance mass spectrometer (FT-ICR-MS), and nuclear magnetic resonance spectrometer, (NMR) but are not limited thereto. According to the present invention, function of the added gene product can be estimated by identifying such a compound that quantitatively changed.

Furthermore, the methods of the present invention for identifying a binding substance include adding at least one gene product to a compound cocktail to react therewith, and detecting changes occurred in the compound cocktail, thereby identifying a binding substance that binds to the gene product. In one embodiment, a method of the present invention for identifying a binding substance comprises the steps of: adding at least one gene product to a compound cocktail; incubating the resultant compound cocktail; removing, from the compound cocktail, the gene product and conjugates between the gene product and the compounds comprised in the cocktail; and detecting changes in compounds comprised in the cocktail, thereby identifying a compound that decreased in quantity in the compound cocktail.

Herein, the compound thus identified by the above process as a compound that decreased in quantity, is very likely a binding substance of the gene product. Thus, the methods of the present invention for identifying a binding substance may further include the step of isolating, from the compound cocktail, conjugates between the gene product and the compound comprised in the compound cocktail, thereby identifying the compound bound to the gene product.

According to the present invention, there is no limitation on the kits for identifying the function of gene products, so long as they comprise the compound cocktail. For example, the kits may additionally comprise: a buffer to be added to the gene product or compound cocktail; a standard substance known to bind to a specific compound in the compound cocktail; a reaction vessel; a reagent for detecting a change in the compound; and an instruction manual. Using the kits of the present invention, the function of gene products can be identified by adding at least one gene product to a compound cocktail to react the gene product with the cocktail, followed by detecting changes occurred in the compound cocktail.

According to the present invention, there is no limitation on a kit for identifying a binding substance of a gene product, so long as it comprises a compound cocktail. For example, such kits may comprise: a buffer to be added to the gene product or compound cocktail; a standard substance known to bind to a specific compound in the compound cocktail; a reaction vessel; a reagent for detecting a change that occurred in the compound; and an instruction manual. Using the kits of the present invention, binding substances of gene products can be identified by adding at least one gene product to a compound cocktail to react the gene product with the cocktail, followed by detecting changes occurred in the compound cocktail.

In the present invention, the term "compound cocktail" means a solution containing a wide variety of compounds such as substrates, coenzymes, and ions, which are necessary for a reaction in a certain reaction system to occur; and low-molecular-weight compounds such as products that result from the reaction. The compound cocktail may also be a metabolic compound cocktail or a cell extract.

Glycolytic compound cocktails and TCA cycle compound cocktails can be given as examples of metabolic compound cocktails.

Compounds contained in a metabolic compound cocktail include, for example, those involved in the glycolytic system, TCA cycle, or pentose phosphate cycle. For example, they include fructose-1,6-phosphate, 6-phosphogluconate, 2,3-phosphoglycerate, glucose-1-phosphate, fructose-6-phosphate, glucose-6-phosphate, ribulose-5-phosphate, ribose-5-phosphate, erythrose-4-phosphate, isocitric acid, citric acid, 2-phosphoglycerate, 3-phosphoglycerate, cis-aconitic acid, phosphoenolpyruvic acid, succinic acid, fumaric acid, lactic acid, and pyruvic acid, but are not limited thereto. For example, the compounds may be any, including amino acids, terpenes, alkaloids, and nucleic acids.

In addition, the cell extract of the present invention includes, for example, bacterial cell extracts, yeast cell extracts, and mammalian tissue extracts (such as brain cell extracts).

Further, the compound cocktail of the present invention may include any substance, so long as it comprises factors necessary for the reaction in which the gene product is involved. For example, the compound cocktail may comprise factors necessary for the gene product to function, including substrates involved in the reaction with the gene product, coenzymes such as ATP and NADH, trace metal elements such as Fe and Mn, various inorganic salts such as MgCl₂, MgSO₄, NaCl, and KCl. The compound cocktail may also be a reconstituted mixture in which necessary factors are added to the buffer.

Specifically, the present invention provides:
[1] a method for identifying a gene product function, wherein the method comprises:
   adding at least one gene product to a compound cocktail; reacting the mixture; detecting a change that occurred in the compound cocktail; and thereby identifying the function of the gene product;
[2] the method of [1], wherein the at least one gene product is obtained by expressing at least one gene encoding the gene product;
[3] the method of [1] or [2], wherein the compound cocktail is a metabolic compound cocktail;
[4] the method of [3], wherein the metabolic compound cocktail comprises a compound(s) selected from the group consisting of fructose-1,6-phosphate, 6-phosphogluconate, 2,3-phosphoglycerate, glucose-1-phosphate, fructose-6-phosphate, glucose-6-phosphate, ribulose-5-phosphate, ribose-5-phosphate, erythrose-4-phosphate, isocitric acid, citric acid, 2-phosphoglycerate, 3-phosphoglycerate, cis-aconitic acid, phosphoenolpyruvic acid, succinic acid, fumaric acid, lactic acid, and pyruvic acid;
[5] the gene product function identification method of [1] or [2], wherein the compound cocktail is a cell extract;
[6] the method of any one of [1] to [5], wherein the change is detected using a capillary electrophoresis-mass spectrometer (CE/MS);
[7] a method for identifying a binding substance to a gene product, wherein the method comprises: adding at least one gene product to a compound cocktail; reacting the mixture; detecting a change that occurred in the compound cocktail; and thereby identifying a binding substance of the gene product;
[8] a kit for identifying a gene product function, wherein the kit comprises a compound cocktail, and the function is identified by adding at least one gene product to the compound cocktail, reacting the mixture, and detecting a change occurred in the compound cocktail;
[9] the kit of [8], wherein the compound cocktail is a metabolic compound cocktail;
[10] the kit of [9], wherein the metabolic compound cocktail comprises a compound(s) selected from the group consisting of fructose-1,6-phosphate, 6-phosphogluconate, 2,3-phosphoglycerate, glucose-1-phosphate, fructose-6-phosphate, glucose-6-phosphate, ribulose-5-phosphate, ribose-5-phosphate, erythrose-4-phosphate, isocitric acid, citric acid, 2-phosphoglycerate, 3-phosphoglycerate, cis-aconitic acid, phosphoenolpyruvic acid, succinic acid, fumaric acid, lactic acid, and pyruvic acid;
[11] the kit of [8], wherein the compound cocktail is a cell extract; and
[12] a kit for identifying a binding substance of a gene product, wherein the kit comprises a compound cocktail, and the binding substance is identified by adding at least one gene product to the compound cocktail, reacting the mixture and detecting a change that occurred in the compound cocktail.

### Brief Description of the Drawings

Fig. 1 is a graph showing the result of measuring each metabolite concentration in a standard solution containing 19 different substrates (100 µM each), determined using CE/MS in the Example of the present invention.
Fig. 2 is a graph showing the result of measuring each metabolite concentration compared with the control experiment determined using CE/MS after addition of a functionally unknown gene product to a standard solution containing 19 substrates, and reacting the product with the standard solution (see the Example).

### Best Mode for Carrying Out the Invention

Hereinbelow, modes for carrying out the present invention consummated based on the above findings will be specifically described using Examples. If there is no explanation in the modes and Examples, methods described in standard protocols such as Molecular Cloning: a laboratory manual (3rd edition), J. Sambrook & D. W. Russell (Ed.), Cold Spring Harbor Laboratory Press, Cold Spring Harbor, New York (2001); Current Protocols in Molecular Biology, F. M. Ausubel, R. Brent, R. E. Kingston, D. D. Moore, J.G Seidman, J. A. Smith, K. Struhl (Ed.), John Wiley & Sons Ltd., or modified or altered methods of these methods may be used. In addition, when commercially available reagent kits or assay instruments are used, protocols attached thereto are used unless otherwise stated.

In addition, since the objectives, characteristics, advantages, and ideas of the present invention will be clear to those skilled in the art by the descriptions of the present specification, one skilled in the art can easily reproduce the present invention from these descriptions. The modes for carrying out the invention and the specific Example below show preferable embodiments of the present invention to exemplify or illustrate the present invention, but the invention is not limited thereto. It is obvious to those skilled in the art that within the range of the present invention's purpose and scope disclosed herein, a variety of alterations and modifications can be achieved based on the descriptions of the present specification.

### Methods for identifying the function of gene products

### (1) Production of gene products

First of all, gene products, i.e. proteins, whose function are to be identified are produced.

The gene products may be derived from any of living things, tissues, organs, cells, and such, without limitation.

The target gene products may be obtained either by *in vitro* or *in vivo* synthesis or by purifying, from cells, gene products originally present in living things (endogenous gene products). When synthesizing the gene products without having the genes encoding the gene products in hand, even though chemical synthesis is possible, it is preferable to synthesize the gene products *in-vivo* or *in-vitro* after first obtaining the gene.

Methods for obtaining genes may include: determining the nucleotide sequences encoding gene products from the amino acid sequences; and chemically synthesizing nucleotides having the nucleotide sequences. When genes are long, it is preferable to clone the cDNAs through PCR or screening of a cDNA library.

The cloned genes are inserted into an expression vector, followed by expression in *Escherichia coli* cells or in cultured cells, and then purification of the resulting gene products. Alternatively, the gene products may be synthesized using *in-vitro* transcription and *in-vitro* translation systems, and then purified.

Purification levels of the gene products may be appropriately adjusted depending on the gene product type. For example, when a membrane protein is to be used, following crude isolation from cells, the membrane protein may be used in a membrane-embedded form to maintain its function. However, to avoid contaminating the compound cocktail, the gene products are preferably highly purified.

### (2) Addition of gene products to the compound cocktail

The gene product(s) obtained as in (1) is added to the compound cocktail.

At least one gene product is added to the compound cocktail. For example, when interactions of multiple proteins are known to be involved in the expression of function, such multiple proteins may be added to one compound cocktail.

The gene products may be added to the compound cocktail at concentrations ranging from 10⁻⁸ µg/ml to 10³ µg/ml.

When a gene product involved in a certain reaction system is added to a compound cocktail that covers the reaction system, the compound cocktail may comprise factors necessary for the functioning of the gene product, including substrates, coenzymes such as ATP and NADH, trace metal elements such as Fe and Mn, and various inorganic salts such as MgCl₂, NaCl, and KCl. Alternatively, these factors may not be comprised in the compound cocktail, and may be appropriately added to the compound cocktail just before the beginning of the reaction. The reaction solution is a buffered solution preferably near the neutral pH range, for example, 6 to 8.

In addition, the compound cocktail may be either a reconstituted mixture to which necessary factors have been added to a buffer solution, or a cell extract.

### (3) Reaction of gene products with compound cocktail

Next, the gene products are reacted with the compound cocktail.

The reaction of the gene products with the compound cocktail is carried out under appropriate time and temperature conditions. Generally, incubation at 37°C for 30 to 120 min is preferable.

The type of reaction that occurs differs depending on the type of gene product(s) added; that is, an enzymatic reaction would occur when the gene product is an enzyme, and a binding reaction would occur when the gene product is a receptor.

### (4) Detection of changes in the compound cocktail

After termination of the reaction, changes occurred in the compound cocktail is detected.

Before the detection, contaminants (for example, proteins added as the gene product) are removed in advance. For removing contaminants, any method for purifying substances including, ultrafiltration, column chromatography, salting out, solvent precipitation, solvent extraction, distillation, immunoprecipitation, SDS-polyacrylamide gel electrophoresis, isoelectric point electrophoresis, dialysis, and recrystallization may be used.

For the detection of changes occurred in compounds comprised in the compound cocktail, analytical instruments including capillary electrophoresis-mass spectrometer (CE/MS), liquid chromatography-mass spectrometer (LC/MS), gas chromatography-mass spectrometer (GC/MS), Fourier transform ion cyclotron resonance mass spectrometer (FT-ICR-MS), and nuclear magnetic resonance spectrometer (NMR), may be used. These instruments enable simultaneous detection of changes in quantity of each factor contained in the compound cocktail.

### (5) Estimation of the function of gene products

Finally, the quantitatively changed compound is identified, and the function of the gene products added to the cocktail is estimated.

When using a reconstituted mixture prepared by adding compounds (factors) with known components as the compound cocktail, the quantitatively changed compound can be identified by assaying the reconstituted mixture beforehand under the same conditions to find out the mass number, detection time, peak area, and such of each factor. When the components of the factors contained in the compound cocktail is unknown, for example, when a cell extract is used as the compound cocktail, the quantitatively changed compound may be identified, using standard analytical methods, based on: structural information obtained by a mass spectrometer (MS/MS) or NMR, a composition formula obtained from the precise mass number obtained by time-of-flight mass spectrometer (TOFMS); detection time and metabolite databases obtained by CE/MS or LC/MS, and such.

From the quantitatively changed compound thus identified, function of the added gene products may be estimated.

### Application examples of the present invention

One typical example of the compound cocktail is a metabolic compound cocktail. More specifically, a glycolytic compound cocktail or a TCA cycle compound cocktail may be used as the metabolic compound cocktail. Metabolic substances involved in such metabolic systems are now commercially available, allowing addition of all of the metabolic substances involved in the metabolic system of interest to the compound cocktail. Compounds comprised in a metabolic compound cocktail include fructose-1,6-phosphate, 6-phosphogluconate, 2,3-phosphoglycerate, glucose-1-phosphate, fructose-6-phosphate, glucose-6-phosphate, ribulose-5-phosphate, ribose-5-phosphate, erythrose-4-phosphate, isocitric acid, citric acid, 2-phosphoglycerate, 3-phosphoglycerate, cis-aconitic acid, phosphoenolpyruvic acid, succinic acid, fumaric acid, lactic acid, and pyruvic acid, but are not limited thereto. When the gene product of interest is an enzyme, the substrate is converted into the product if the gene product is functionally active. Thus, addition of the gene product to a metabolic compound cocktail in which the enzyme works will result in, after the reaction, a decrease in the substrate amount and an increase in the product amount. Using this principle, many kinds of metabolic compound cocktails are prepared, a functionally unknown gene product is added thereto, and changes in the amount of a low-molecular-weight compound contained in each metabolic compound cocktail are measured. If compound A is decreased and compound B is increased, the gene product is suggested to have the ability to convert A to B in the metabolic system.

When the gene product of interest is a protein that binds to a low-molecular-weight compound, the gene product will bind to the low-molecular-weight compound (the corresponding binding substance) if the compound is present in the compound cocktail, resulting in a decrease of free binding substance after the reaction. Thus, it is highly possible that the compound of which the amount decreases after the reaction is a binding substance to the gene product. For example, using this system, it may be possible to verify a low-molecular-weight ligand that binds to a certain receptor.

Multiple gene products may also be added. For example, such multiple gene products known to genetically interact with each other or those known to biochemically bind to each other may also be added. When these gene products form a quaternary structure to cause an enzyme activity or a binding activity, changes in the amount of factors in the compound cocktail occur only when the products are added together. In addition, when each of these multiple gene products is involved in independent reactions, the number of factors that change after the reaction are not only two, but may also be three or more.

The compound cocktail used may also be a cell extract. Cell extracts include bacterial cell extracts, yeast cell extracts, mammalian tissue extracts (for example, brain cell extracts), but are not limited thereto. Any extract may be used without limitation so long as it comprises factors necessary forthe reaction in which the gene product involves.

In addition, all prior art literatures cited herein are incorporated by reference into the present specification.

### Examples

Hereinbelow, an experiment example in which the function of a functionally unknown *Escherichia coli* gene product is identified will be specifically described as an Example, but the present invention is not to be construed as being limited thereto.

As the compound cocktail, 100 µl of HEPES buffer (5 mM, pH7.5) comprising 19 different substrate compounds involved in the glycolytic system, TCA cycle, or pentose phosphate cycle (fructose-1,6-phosphate (F16P), 6-phosphogluconate (6PG), 2,3-phosphoglycerate (23DPG), glucose-1-phosphate (G1P), fructose-6-phosphate (F6P), glucose-6-phosphate (G6P), ribulose-5-phosphate (Ribulose5P), ribose-5-phosphate (Ribose5P), erythrose-4-phosphate (Erythrose4P), isocitric acid (iso-Citrate), citric acid (Citrate), 2-phosphoglycerate, 3-phosphoglycerate (2PG/3PG), cis-aconitic acid (cis-Aconitate), phosphoenolpyruvic acid (PEP), succinic acid (Succinate), fumaric acid (Fumarate), lactic acid (Lactate), and pyruvic acid (Pyruvate)) (100 µM each; the final concentration is shown in all parentheses hereafter), supplemented with NADH (500 µM), MgSO₄ (10 mM), and KCl (10 mM) was used.

Moreover, a gene suggested to be involved in the above metabolic pathway was cloned into a prokaryotic expression vector having a His-tag sequence, followed by induction of gene expression with IPTG. Then, *Escherichia coli* cells were harvested, disrupted by sonication. The resulting cell extract was loaded onto a cobalt column to bind the protein via His-tag. After washing the column with 20 mM imidazole, the protein was recovered from the column with a HEPES elution buffer (150 mM imidazole-300 mM NaCl-50 mM HEPES, pH7.0). Electrophoresis was carried out to confirm the purity of the protein, and the eluate comprising the target protein was desalted, concentrated, and stored at -20°C until use.

One µg of the purified protein was added to the above compound cocktail and incubated at 37°C for 30 min. Immediately thereafter, the protein was removed by ultrafiltration and the reaction mixture was analyzed using a capillary electrophoresis-mass spectrometer (CE/MS). For a control experiment, the same reaction was carried out without adding the above-described gene product.

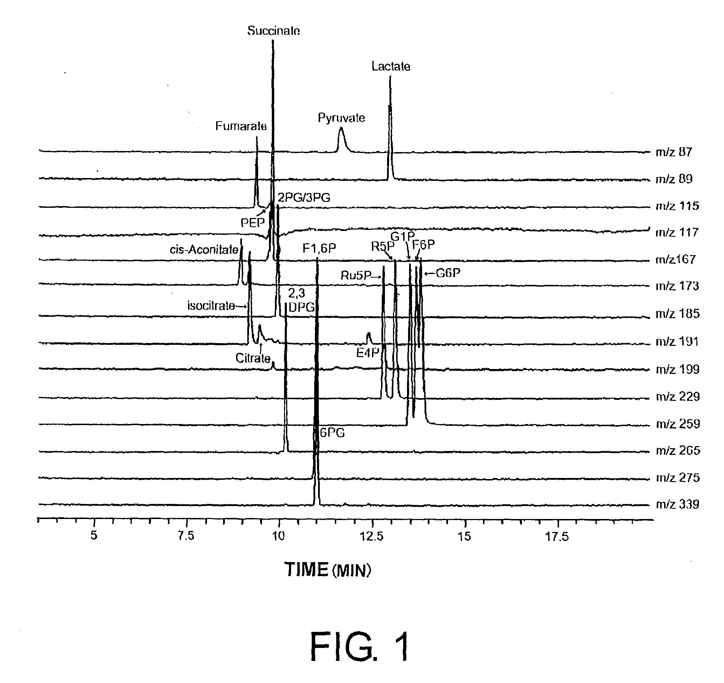

CE/MS was used according to the instructions described in documents (Japanese Patent No. 3341765 and Soga T, et al., Anal. Chem., 74, 2233-2239 (2002)). Measurement conditions for CE/MS are mentioned below. A SMILE (+) capillary with an internal diameter of 50 µm, an external diameter of 350 µm, and a length of 90 cm was used as capillary, and 50 mM ammonium acetate (pH8.5) was used as electrophoresis buffer. A -30 kV voltage was applied to platinum electrode from a high voltage power supply and the capillary temperature was set at 20°C. The sample was injected at 50 mbar for 30 sec using the pressure method. The Electrospray Ionization Mass Spectrometer (ESI-MS) was used as the mass spectrometer. The negative ion mode was used to selectively introduce anions into the MS by using the MS-side electrode as cathode. The capillary voltage applied to the capillary in the negative ion mode was set at 4,000 V. The fragmentor voltage applied to the cone section was set at 100 V, to allow generation of fragment ions (fragments of the substance) through ion acceleration and collision with nitrogen gas. Nitrogen gas was used for the drying gas that is used for evaporating the solvent from CE and the gas temperature was set at 300°C for the analysis. As a sheath liquid, a 50% methanol solution containing 5 mM ammonium acetate was used and injected at a flow rate of 10 µl/min. For reference, Fig. 1 shows the result of the measurement of standard solution containing 19 different substrates (100 µM each) under the above-described conditions. Each compound can be detected as its own mass number (m/z).

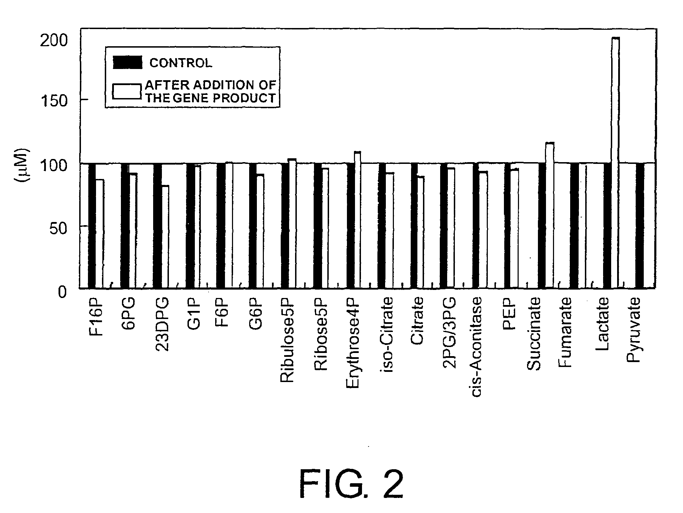

Fig. 2 shows the actual experiment result obtained by adding the above-described gene product to this standard solution containing 19 different substrates and reacting this when compared to the control experiment. A significant decrease in the concentration of pyruvic acid accompanied by an increase in the concentration of lactic acid was observed when the gene product was added, compared with the control experiment conducted without adding the gene product. This result suggests that the added gene product has an activity to convert pyruvic acid to lactic acid, showing that the gene product is lactate dehydrogenase.

### Industrial Applicability

The present invention can provide methods and kits for identifying the function of a functionally unknown gene product widely applicable to a wide variety of organic species. The present invention also can provide methods and kits for identifying a substance binding to the functionally unknown gene product.

## Claims

1. A method for identifying a gene product function, wherein the method comprises: adding at least one gene product to a compound cocktail; reacting the mixture; detecting a change that occurred in the compound cocktail; and thereby identifying the function of the gene product.

2. The method of claim 1, wherein the at least one gene product is obtained by expressing at least one gene encoding the gene product.

3. The method of claim 1 or 2, wherein the compound cocktail is a metabolic compound cocktail.

4. The method of claim 3, wherein the metabolic compound cocktail comprises a compound(s) selected from the group consisting of fructose-1,6-phosphate, 6-phosphogluconate, 2,3-phosphoglycerate, glucose-1-phosphate, fructose-6-phosphate, glucose-6-phosphate, ribulose-5-phosphate, ribose-5-phosphate, erythrose-4-phosphate, isocitric acid, citric acid, 2-phosphoglycerate, 3-phosphoglycerate, cis-aconitic acid, phosphoenolpyruvic acid, succinic acid, fumaric acid, lactic acid, and pyruvic acid.

5. The gene product function identification method of claim 1 or 2, wherein the compound cocktail is a cell extract.

6. The method of any one of claims 1 to 5, wherein the change is detected using a capillary electrophoresis-mass spectrometer (CE/MS).

7. A method for identifying a binding substance to a gene product, wherein the method comprises: adding at least one gene product to a compound cocktail; reacting the mixture; detecting a change that occurred in the compound cocktail; and thereby identifying a binding substance of the gene product.

8. A kit for identifying a gene product function, wherein the kit comprises a compound cocktail, and the function is identified by adding at least one gene product to the compound cocktail, reacting the mixture, and detecting a change occurred in the compound cocktail.

9. The kit of claim 8, wherein the compound cocktail is a metabolic compound cocktail.

10. The kit of claim 9, wherein the metabolic compound cocktail comprises a compound(s) selected from the group consisting of fructose-1,6-phosphate, 6-phosphogluconate, 2,3-phosphoglycerate, glucose-1-phosphate, fructose-6-phosphate, glucose-6-phosphate, ribulose-5-phosphate, ribose-5-phosphate, erythrose-4-phosphate, isocitric acid, citric acid, 2-phosphoglycerate, 3-phosphoglycerate, cis-aconitic acid, phosphoenolpyruvic acid, succinic acid, fumaric acid, lactic acid, and pyruvic acid.

11. The kit of claim 8, wherein the compound cocktail is a cell extract.

12. A kit for identifying a binding substance of a gene product, wherein the kit comprises a compound cocktail, and the binding substance is identified by adding at least one gene product to the compound cocktail, reacting the mixture and detecting a change that occurred in the compound cocktail.
